# EUROPEAN PATENT SPECIFICATION

(11) **EP 1 833 521 B1**
(45) Date of publication and mention of the grant of the patent: **05.08.2009**
(21) Application number: 05820526.1
(22) Date of filing: 22.12.2005
(51) Int. Cl.: A61L 15/60

(54) **SUPERABSORBENT POLYMER PARTICLES HAVING A REDUCED AMOUNT OF FINE-SIZED PARTICLES, AND METHODS OF MANUFACTURING THE SAME**
SUPERSAUGFÄHIGE POLYMERTEILCHEN MIT EINER VERRINGERTEN MENGE AN FEINEN TEILCHEN UND HERSTELLUNGSVERFAHREN DAFÜR
PARTICULES DE POLYMERE SUPER-ABSORBANT AYANT UNE QUANTITE REDUITE DE PARTICULES DE FAIBLE TAILLE, ET PROCEDES POUR LES PREPARER

(30) Priority: 30.12.2004 US 640321 P
(43) Date of publication of application: 19.09.2007
(73) Proprietor: BASF SE, 67056 Ludwigshafen (DE)
(72) Inventor: WOODRUM, Guy T., Suffolk, VA 23435 (US); AZAD, Michael M., Charlotte, NC 28277 (US); HERFERT, Norbert, Charlotte, NC 28226-7200 (US)
(86) International application number: PCT/EP2005/013870
(87) International publication number: WO 2006/069732

(56) References cited:
- US-A- 5 681 878
- US-A- 5 731 365
- US-A- 5 840 321
- US-A- 6 124 391

## Description

### FIELD OF THE INVENTION

The present invention relates to coated superabsorbent polymer particles having a reduced amount of fine-sized particles. The present invention also relates to methods of manufacturing superabsorbent polymer particles using a film-forming polymer or a wax to reduce the amount of fine-sized superabsorbent polymer particles. The present invention also relates to the use of the superabsorbent polymer particles in articles, such as diapers, catamenial devices, and wound dressings.

### BACKGROUND OF THE INVENTION

Water-absorbing resins are widely used in sanitary goods, hygienic goods, wiping cloths, water-retaining agents, dehydrating agents, sludge coagulants, disposable towels and bath mats, disposable door mats, thickening agents, disposable litter mats for pets, condensation-preventing agents, and release control agents for various chemicals. Water-absorbing resins are available in a variety of chemical forms, including substituted and unsubstituted natural and synthetic polymers, such as hydrolysis products of starch acrylonitrile graft polymers, carboxymethylcellulose, crosslinked polyacrylates, sulfonated polystyrenes, hydrolyzed polyacrylamides, polyvinyl alcohols, polyethylene oxides, polyvinylpyrrolidones, and polyacrylonitriles. The most commonly used polymer for absorbing electrolyte-containing aqueous fluids, such as urine, is neutralized polyacrylic acid, i.e., containing about 50% to 100%, neutralized carboxyl groups.

Such water-absorbing resins are termed "superabsorbent polymers," or SAPs, and typically are lightly crosslinked hydrophilic polymers. SAPs are generally discussed in Goldman et al. U.S. Patent Nos. 5,669,894 and 5,599,335, each incorporated herein by reference. SAPs can differ in their chemical identity, but all SAPs are capable of absorbing and retaining amounts of aqueous fluids equivalent to many times their own weight, even under moderate pressure. For example, SAPs can absorb one hundred times their own weight, or more, of distilled water. The ability to absorb aqueous fluids under a confining pressure is an important requirement for an SAP used in a hygienic article, such as a diaper.

As used herein, the term "SAP particles" refers to superabsorbent polymer particles in the dry state, i.e., particles containing from no water up to an amount of water less than the weight of the particles. The term "particles" refers to granules, fibers, flakes, spheres, powders, platelets, and other shapes and forms known to persons skilled in the art of superabsorbent polymers. The terms "SAP gel" and "SAP hydrogel" refer to superabsorbent polymer particles in the hydrated state, i.e., particles that have absorbed at least their weight in water, and typically several times their weight in water. The term "coated SAP particles" refers to particles of the present invention, i.e., SAP particles having a water-insoluble polymer or wax coating.

The terms "fine-sized SAP particles" and "SAP fines" refer to SAP particles having a particle diameter of about 100 microns or less. In the production of SAP particles, a significant portion of SAP fines, e.g., about 10 percent by weight, can be generated. The present invention is directed to substantially reducing the amount of SAP fines generated during manufacture of SAP particles.

The term "surface crosslinked" refers to an SAP particle having its molecular chains present in the vicinity of the particle surface crosslinked by a compound applied to the surface of the particle. The term "surface crosslinking" means that the level of functional crosslinks in the vicinity of the surface of the SAP particle generally is higher than the level of functional crosslinks in the interior of the SAP particle. As used herein, "surface" describes the outer-facing boundaries of the particle. For porous SAP particles, exposed internal surfaces also are included in the definition of surface.

The term "polymer coating" or "wax coating" refers to a coating on the surface of an SAP particle comprising a nonreactive, water-insoluble polymer and/or wax. The film-forming polymer that coats the surface of the SAP particle is different from the polymeric material of the SAP particle, and typically does not function as an SAP. The polymer and/or wax coating does not adversely affect the absorption profile of the SAP particles, and reduces the amount of SAP fines generated during SAP particle manufacture.

SAP particles can differ in ease and cost of manufacture, chemical identity, physical properties, rate of water absorption, and degree of water absorption and retention, thus making the ideal water-absorbent resin a difficult composition to design. Therefore, extensive research and development has been directed to providing a method of increasing the fluid absorption properties of SAP particles. This is a difficult goal to achieve because improving one desirable property of an SAP particle often adversely affects another desirable property of the SAP particle. For example, absorptivity and gel permeability are conflicting properties. Therefore, a balanced relation between absorptivity and gel permeability is desired in order to provide sufficient liquid absorption, liquid transport, and dryness of the diaper and the skin when using SAP particles in a diaper.

In this regard, not only is the ability of the SAP particles to retain a liquid under subsequent pressure an important property, but absorption of a liquid against a simultaneously acting pressure, i.e., during liquid absorption, also is important. This is the case in practice when a child or adult sits or lies on a sanitary article, or when shear forces are acting on the sanitary article, e.g., leg movements. This absorption property is referred to as absorption under load.

The current trend in the hygiene sector, e.g., in diaper design, is toward ever thinner core constructions having a reduced cellulose fiber content and an increased SAP content. This is an especially important trend in baby diapers and adult incontinence products.

This trend has substantially changed the performance profile required of SAPs. Whereas SAP development initially was focused on very high absorption and swellability, it subsequently was determined that an ability of SAP particles to transmit and distribute a fluid both into the particle and through a bed of SAP particles also is of major importance. Conventional SAPs undergo great surface swelling when wetted with a fluid, such that transport of the fluid into the particle interior is substantially compromised or completely prevented. Accordingly, a substantial amount of cellulose fibers have been included in a diaper core to quickly absorb the fluid for eventual distribution to the SAP particles, and to physically separate SAP particles in order to prevent fluid transport blockage.

An increased amount of SAP particles per unit area in a hygiene article must not cause the swollen SAP hydrogel particles to form a barrier layer to absorption of a subsequent fluid insult. Therefore, an SAP having good permeability properties ensures optimal utilization of the entire hygiene article. This prevents the phenomenon of gel blocking, which in the extreme case causes the hygiene article to leak. Fluid transmission and distribution, therefore, is of maximum importance with respect to the initial absorption of body fluids.

Fine-sized SAP particles contribute significantly to gel blocking. Therefore, extensive effort has been expended to find methods of reducing the generation of SAP fines during SAP manufacture. The reduction of SAP fines is one method wherein permeability properties of SAP particles can be improved without a concomitant reduction in absorption properties.

As previously stated, SAPs typically are based on acrylic acid and/or a salt thereof. The SAP particles are prepared by polymerizing acrylic acid and/or a salt thereof in the presence of an internal crosslinking agent. In general, a monomer solution containing acrylic acid and/or an acrylic acid salt, plus an internal crosslinking agent, is polymerized to form a hydrogel. The hydrogel then is chopped and dried, followed by milling and sieving.

Sieving separates fine-sized SAP particles from SAP particles having the desired particle size. The fine-sized SAP particles have a particle size of less than about 100 µm, and are separated because SAP fines adversely affect SAP performance in an absorbent article. In particular, commercial applications of SAP fines are limited by the small particle size which typically results in handling problems, such as dusting, and performance problems, such as gel blocking.

Initially, manufacturers used the entire SAP product, including fines, in absorbent products. It was discovered, however, that the inclusion of SAP fines lowered product performance. The difficulty occurs when the fine particles are contacted with an aqueous fluid, which results in a "gel blocking" phenomenon. Upon hydration of a tightly packed mass of SAP fines, only the outside layer is wetted because the fines form a dense network such that neither capillary action nor diffusion permits penetration of the fluid into uniform contact with the interior particles. The result is a substantially reduced overall capacity of the SAP to absorb and hold aqueous fluids.

SAP fines also produce a dusting problem. Fine SAP dust having a particle size of less than about 10 µm is undesirable for inhalation toxicity reasons, and SAP fines smaller than 100 µm cause visually detectable dusting that lead to handling problems in production and processing operations.

The sieved fractions of SAP particles having a particle size of about 100 µm or greater are admixed, then typically subjected to a surface crosslinking process, followed by a second sieving operation. The surface-crosslinking process produces additional SAP fines, and the second sieving operation separates additional SAP fines, i.e., surface-crosslinked SAP fines, also having a particle size of less than about 100 microns.

The separated fine-sized SAP particles, including surface-crosslinked SAP fines, typically are not used in practical applications, and either are discarded or recycled into a wet stage of the SAP manufacturing process such that their polymeric material forms a part of the final dried SAP particles. Simply discarding SAP fines is economically undesirable. However, recycling of SAP fines also is costly. The once or twice (if surface crosslinked) dried SAP fines are reintroduced early into the SAP process for recycling, which hydrates the SAP fines. Consequently, the prior drying steps, including the time required and energy costs, are wasted for the percent of SAP fines that are recycled. In addition, even after recycling, the subsequent drying and sizing steps generate additional SAP fines that again must be reintroduced in the SAP manufacturing process. Accordingly, the recycling of SAP fines is a continuous and costly process.

Directly recycling SAP fines into the SAP manufacturing process also has other disadvantages, for example, providing SAP particles that resist drying or that dry at uneconomical rates. Recycling SAP fines also often fails to provide an SAP particle that resists disintegration during subsequent processing, such as sizing, pneumatic conveying, or surface crosslinking, which are necessary for forming commercial SAP products. The recycling of SAP fines in this manner also is costly, and tends to reduce the gel strength of the final SAP particles.

The present invention is directed to SAP particles, optionally surface crosslinked, that are coated with a nonreactive, film-forming polymer and/or a wax. The methods of manufacturing these coated SAP particles reduce the amount of fine-sized SAP particles that are generated, and, therefore, decrease manufacturing costs by reducing the amount of SAP fines that are recycled.

### SUMMARY OF THE INVENTION

The present invention is directed to SAP particles having a coating comprising a film-forming polymer and/or a wax. More particularly, the present invention is directed to methods of preparing optionally surface-crosslinked SAP particles that generate a reduced amount of fine-sized particles, and to SAP particles having a coating comprising a film-forming polymer and/or a wax.

One aspect of the present invention is to provide methods of manufacturing SAP particles that generate a reduced amount of fine-sized particles, i.e., particles less than 100 microns in size. As a result, recycling of SAP fines is substantially reduced and significant cost savings are achieved.

Another aspect of the present invention is to provide optionally surface-crosslinked SAP particles coated with a film-forming polymer and/or wax. Coating of the SAP particles with a film-forming polymer and/or wax, and optional surface crosslinking, can be performed simultaneously or sequentially. In the absence of a surface crosslinking process, the film-forming polymer and/or wax is applied to SAP hydrogel particles after a chopping, i.e., sizing, step and prior to or during a drying step.

Still another aspect of the present invention is to prepare coated SAP particles of the present invention by applying less than about 0.1% of a film-forming polymer, less than 0.05% of a wax, or both, by weight of the SAP particles, to surfaces of the SAP particles, followed by heating the resulting SAP particles at about 70°C to 175°C for about 5 to about 90 minutes.

Another aspect of the present invention is to provide a method of manufacturing SAP particles wherein the method generates less than 2%, by weight, particles having a particle size of 100 microns or less.

Yet another aspect of the present invention is to provide absorbent hygiene articles, such as diapers, having a core comprising coated SAP particles of the present invention.

Another aspect of the present invention is to provide absorbent hygiene articles having a core containing a relatively high concentration of coated SAP particles of the present invention.

These and other aspects and advantages of the present invention will become apparent from the following detailed description of the preferred embodiments.

### DETAILED DESCRIPTION OF THE PREFERRED EMBODIMENTS

The present invention is directed to SAP particles coated with a film-forming polymer and/or a wax. The polymer or wax is applied to SAP particles or a comminuted SAP hydrogel during the manufacturing process, which reduces the amount of fine-sized SAP particles generated during subsequent processing. Accordingly, a reduced amount of SAP fines are recycled through the SAP manufacturing process, which results in a substantial cost savings and SAP particles having an improved absorption profile.

SAPs for use in personal care products to absorb body fluids are well known. SAP particles typically are polymers of unsaturated carboxylic acids or derivatives thereof. These polymers are rendered water insoluble, but water swellable, by crosslinking the polymer with a di- or polyfunctional internal crosslinking agent. These internally crosslinked polymers are at least partially neutralized and contain pendant anionic carboxyl groups on the polymer backbone that enable the polymer to absorb aqueous fluids, such as body fluids.

SAPs are manufactured by known polymerization techniques, preferably by polymerization in aqueous solution by gel polymerization. The products of this polymerization process are aqueous polymer gels, i.e., SAP hydrogels, that are reduced in size to small particles by mechanical forces, then dried using drying procedures and apparatus known in the art. The drying process is followed by pulverization of the resulting SAP particles to the desired particle size.

To improve the fluid absorption profile, SAP particles are optimized with respect to one or more of absorption capacity, absorption rate, acquisition time, gel strength, and/or permeability. Optimization allows a reduction in the amount of cellulosic fiber in a hygienic article, which results in a thinner article. However, it is difficult to impossible to maximize all of these absorption profile properties simultaneously.

One method of optimizing the fluid absorption profile of SAP particles is to provide SAP particles of a predetermined particle size distribution. In particular, particles too small in size, after absorbing a fluid and swelling, and can block the absorption of further fluid. Particles too large in size have a reduced surface area which decreases the rate of absorption.

Therefore, the particle size distribution of SAP particles is such that fluid permeability, absorption, and retention by the SAP particles is maximized. Any subsequent process that reduces SAP particle size should be avoided. In particular, fine-sized SAP particles are prone to gel blocking, which adversely affects absorption of an aqueous fluid by the SAP particles.

Invariably, a substantial amount of fine-sized SAP particles, i.e., particles less than about 100 microns in size, are produced during SAP manufacture. These fine-sized SAP particles adversely affect SAP performance in a majority of practical applications, and are separated from SAP particles of desired size. It is economically disadvantageous to discard the fine-sized SAP particles, therefore, the particles are recycled into an early stage of the SAP manufacturing process. This recycling step also is costly because the previously dried SAP fines are rehydrated, then dried again. In addition, each SAP manufacturing process generates additional SAP fines, so the recycling process is constant.

The present invention is directed to abating the problem of fine-sized SAP particles generated during the manufacturing process. The problem of SAP fines is abated because of the polymer and/or wax coating applied to the surfaces of the SAP particles. More particularly, the present method of manufacturing SAP particles substantially reduces the amount of SAP fines, and the SAP particles maintain the conflicting fluid absorption properties of a high centrifuge retention capacity (CRC), absorption under load (AUL), and an excellent permeability.

In order to use an increased amount of SAP particles, and a decreased amount of cellulose, in personal care products, it is important to maintain a high liquid permeability. In particular, the permeability of an SAP particle hydrogel layer formed by swelling in the presence of a body fluid is very important to overcome the problem of leakage from the product. Fine-sized SAP particles adversely affect permeability, and a lack of permeability directly impacts the ability of SAP particle layers to acquire and distribute body fluids.

Numerous approaches to reduce the amount of fine-sized SAP particles generated during the manufacturing process have been proposed. These prior methods include using additional crosslinking agent or polymerization initiator near the end of SAP manufacture. Another approach is disclosed in Japanese Patent Publication 622411972, which discloses coating SAP particle surfaces with a polymer emulsion, then over-coating with a coarse emulsion. The second coating provides a waterproof coating, i.e., a low water penetration of 2.1%.

U.S. Patent No. 5,731,365 and U.S. Patent No. 5,840,321 address the dusting problem generated by a high fine-sized particle in SAP content by coating the SAP with a non-reactive, water-insoluble film-forming polymer that is employed in amounts ranging from 0.1 wt.-% to 40 wt.-% and preferably 0.5 wt.-% to 20 wt.-%, based on the total SAP weight, or by coating the SAP with a non-reactive, water-insoluble wax that is employed in amounts ranging from 0.05 wt.-% to 0.2 wt.-%, preferably 0.1 wt.-% to 0.95 wt.-% and most preferably 0.2 to 0.4 wt.-%, based on the total SAP weight.

Other patents directed to reducing the amount of, or adverse effects of, SAP fines include U.S. Patent No. 5,478,879; EP 0 401 044; U.S. Patent No. 4,970,267; and U.S. Patent No. 5,064,582. How-5 ever, these and other approaches continue to generate a significant amount of SAP particles too small in size for current market applications. Using these approaches, substantial amounts of SAP fines still are generated during subsequent processing steps when the SAP particles, before or after surface crosslinking, are ground and sieved to meet desired particle size specifications.

In accordance with the present invention, optionally surface-crosslinked SAP particles coated with a polymer and/or wax are disclosed. The present SAP particles comprise a base polymer. The base polymer can be a homopolymer or a copolymer. The identity of the base polymer is not limited as long as the polymer is an anionic polymer, i.e., contains pendant acid moieties, and is capable of swelling and absorbing at least ten times its weight in water, when in a neutralized form. Preferred base polymers are crosslinked polymers having acid groups that are at least partially in the form of a salt, generally an alkali metal or ammonium salt.

The base polymer typically has at least about 25 mol% of the pendant acid moieties, i.e., carboxylic acid moieties, present in a neutralized form. Preferably, the base polymer has greater than 25 and up to about 100 mol%, and more preferably about 50 up to about 100 mol%, of the pendant acid moieties present in a neutralized form. The base polymer, therefore, has a degree of neutralization (DN) of at least 25 to about 100.

The SAP is a lightly crosslinked polymer capable of absorbing several times its own weight in water and/or saline. SAP particles can be made by any conventional process for preparing superabsorbent polymers and are well known to those skilled in the art. One process for preparing SAP particles is a solution polymerization method described in U.S. Patent Nos. 4,076,663; 4,286,082; 4,654,039; and 5,145,906, each incorporated herein by reference. Another process is an inverse suspension polymerization method described in U.S. Patent Nos. 4,340,706; 4,497,930; 4,666,975; 4,507,438; and 4,683,274, each incorporated herein by reference. Each of these processes yields a substantial amount of fine-sized SAP particles that require recycling.

SAP particles are prepared from one or more monoethylenically unsaturated compound having at least one acid moiety, such as carboxyl, carboxylic acid anhydride, carboxylic acid salt, sulfuric acid, sulfuric acid salt, sulfonic acid, sulfonic acid salt, phosphoric acid, phosphoric acid salt, phosphonic acid, or phosphonic acid salt. SAP particles preferably are prepared from one or more monoethylenically unsaturated, water-soluble carboxyl or carboxylic acid anhydride containing monomer, and the alkali metal and ammonium salts thereof, wherein these monomers preferably comprise 50 to 99.9 mole percent of the base polymer.

The SAP preferably is a lightly crosslinked acrylic resin, such as lightly crosslinked polyacrylic acid. The lightly crosslinked SAP typically is prepared by polymerizing an acidic monomer containing an acyl moiety, e.g., acrylic acid, or a moiety capable of providing an acid group, i.e., acrylonitrile, in the presence of an internal crosslinking agent, i.e., a polyfunctional organic compound. The SAP can contain other copolymerizable units, i.e., other monoethylenically unsaturated comonomers, well known in the art, as long as the base polymer is substantially, i.e., at least 10%, and preferably at least 25%, acidic monomer units, e.g., (meth)acrylic acid. To achieve the full advantage of the present invention, the SAP contains at least 50%, and more preferably, at least 75%, and up to 100%, acidic monomer units. The other copolymerizable units can, for example, help improve the hydrophilicity of the SAP.

Ethylenically unsaturated carboxylic acid and carboxylic acid anhydride monomers useful in an SAP include acrylic acid, methacrylic acid, ethacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, and maleic anhydride.

Ethylenically unsaturated sulfonic and phosphonic acid monomers include aliphatic or aromatic vinyl sulfonic acids, such as vinyl sulfonic acid, allyl sulfonic acid, vinyl toluene sulfonic acid, styrene sulfonic acid, acrylic and methacrylic sulfonic acids, such as sulfoethyl acrylate, sulfoethyl methacrylate, sulfopropyl acrylate, sulfopropyl methacrylate, 2-hydroxy-3-methacryloxypropyl sulfonic acid, 2-acrylamido-2-methylpropane sulfonic acid, vinyl phosphonic acid, allyl phosphonic acid, and mixtures thereof.

Preferred monomers include acrylic acid, methacrylic acid, maleic acid, fumaric acid, maleic anhydride, and the sodium, potassium, and ammonium salts thereof. An especially preferred monomer is acrylic acid.

The SAP can contain additional monoethylenically unsaturated monomers that do not bear a pendant acid group, but are copolymerizable with monomers bearing acid groups. Such compounds include, for example, the amides and nitriles of monoethylenically unsaturated carboxylic acids, for example, acrylamide, methacrylamide, acrylonitrile, and methacrylonitrile. Examples of other suitable comonomers include, but are not limited to, vinyl esters of saturated C₁₋₄ carboxylic acids, such as vinyl formate, vinyl acetate, and vinyl propionate; alkyl vinyl ethers having at least two carbon atoms in the alkyl group, for example, ethyl vinyl ether and butyl vinyl ether; esters of monoethylenically unsaturated C₃₋₁₈ alcohols and acrylic acid, methacrylic acid, or maleic acid; monoesters of maleic acid, for example, methyl hydrogen maleate; acrylic and methacrylic esters of alkoxylated monohydric saturated alcohols, for example, alcohols having 10 to 25 carbon atoms reacted with 2 to 200 moles of ethylene oxide and/or propylene oxide per mole of alcohol; and monoacrylic esters and monomethacrylic esters of polyethylene glycol or polypropylene glycol, the molar masses (Mₙ) of the polyalkylene glycols being up to about 2,000, for example. Further suitable comonomers include, but are not limited to, styrene and alkyl-substituted styrenes, such as ethylstyrene and tert-butylstyrene, and 2-hydroxyethyl acrylate.

Polymerization of the acidic monomers, and any copolymerizable monomers, most commonly is performed by free radical processes in the presence of a polyfunctional organic compound. The base polymers are internally crosslinked to a sufficient extent such that the base polymer is water insoluble. Internal crosslinking, in part, serves to determine the absorption capacity of the base polymer. For use in absorption applications, a base polymer is lightly crosslinked, i.e., has a crosslinking density of less than about 20%, preferably less than about 10%, and most preferably about 0.01% to about 7%.

A crosslinking agent most preferably is used in an amount of less than about 7 wt%, and typically about 0.1 wt% to about 5 wt%, based on the total weight of monomers. Examples of crosslinking polyvinyl monomers include, but are not limited to, polyacrylic (or polymethacrylic) acid esters represented by the following formula (I), and bisacryl-amides represented by the following formula (II): 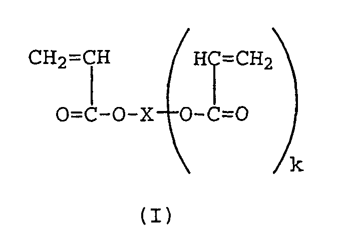 wherein X is ethylene, propylene, trimethylene, cyclohexyl, hexamethylene, 2-hydroxypropylene, - (CH₂CH₂O) ₙCH₂CH₂-, or 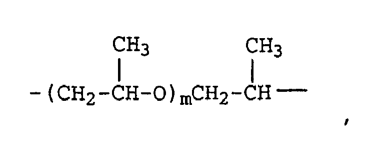 n and m are each an integer 5 to 40, and k is 1 or 2; 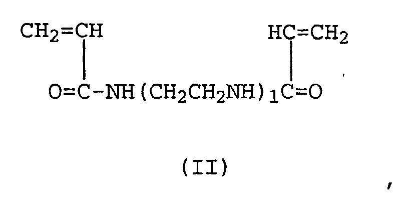 wherein 1 is 2 or 3.

The compounds of formula (I) are prepared by reacting polyols, such as ethylene glycol, propylene glycol, trimethylolpropane, 1,6-hexanediol, glycerin, pentaerythritol, polyethylene glycol, or polypropylene glycol, with acrylic acid or methacrylic acid. The compounds of formula (II) are obtained by reacting polyalkylene polyamines, such as diethylenetriamine and triethylenetetramine, with acrylic acid.

Specific internal crosslinking agents include, but are not limited to, 1,4-butanediol diacrylate, 1,4-butanediol dimethacrylate, 1,3-butylene glycol diacrylate, 1,3-butylene glycol dimethacrylate, diethylene glycol diacrylate, diethylene glycol dimethacrylate, ethoxylated bisphenol A diacrylate, ethoxylated bisphenol A dimethacrylate, ethylene glycol dimethacrylate, 1,6-hexanediol diacrylate, 1,6-hexanediol dimethacrylate, neopentyl glycol dimethacrylate, polyethylene glycol diacrylate, polyethylene glycol dimethacrylate, triethylene glycol diacrylate, triethylene glycol dimethacrylate, tripropylene glycol diacrylate, tetraethylene glycol diacrylate, tetraethylene glycol dimethacrylate, dipentaerythritol pentaacrylate, pentaerythritol tetraacrylate, pentaerythritol triacrylate, trimethylolpropane triacrylate, trimethylolpropane trimethacrylate, tris(2-hydroxyethyl)-isocyanurate triacrylate, ethoxylated trimethylolpropane triacrylate (ETMPTA), e.g., ETMPTA ethyoxylated with 15 moles of ethylene oxide (EO) on average, tris(2-hydroxyethyl)isocyanurate trimethyacrylate, divinyl esters of a polycarboxylic acid, diallyl esters of a polycarboxylic acid, triallyl terephthalate, diallyl maleate, diallyl fumarate, hexamethylenebismale-imide, trivinyl trimellitate, divinyl adipate, diallyl succinate, a divinyl ether of ethylene glycol, cyclopentadiene diacrylate, a tetraallyl ammonium halide, divinyl benzene, divinyl ether, diallyl phthalate, or mixtures thereof. Especially preferred internal crosslinking agents are N,N'-methylenebisacrylamide, N,N'-methylenebismethacrylamide, ethylene glycol dimethacrylate, and trimethylolpropane triacrylate.

The SAP can be any internally-crosslinked polymer having pendant acid moieties that absorbs several times its weight of water in its neutralized form. Examples of SAPs include, but are not limited to, polyacrylic acid, hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly(vinylsulfonic acid), poly(vinylphosphonic acid), poly(vinylphosphoric acid), poly-(vinylsulfuric acid), sulfonated polystyrene, poly-(aspartic acid), poly(lactic acid), and mixtures thereof. A preferred SAP is a homopolymer or copolymer of acrylic acid or methacrylic acid.

The free radical polymerization is initiated by an initiator or by electron beams acting on a polymerizable aqueous mixture. Polymerization also can be initiated in the absence of such initiators by the action of high energy radiation in the presence of photoinitiators.

Useful polymerization initiators include, but are not limited to, compounds that decompose into free radicals under polymerization conditions, for example, peroxides, hydroperoxides, persulfates, azo compounds, and redox catalysts. Water-soluble initiators are preferred. In some cases, mixtures of different polymerization initiators are used, for example, mixtures of hydrogen peroxide and sodium peroxodisulfate or potassium peroxodisulfate. Mixtures of hydrogen peroxide and sodium peroxodisulfate can be in any proportion.

Examples of suitable organic peroxides include, but are not limited to, acetylacetone peroxide, methyl ethyl ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, di(2-ethylhexyl) peroxydicarbonate, dicyclohexyl peroxydicarbonate, di(4-tert-butylcyclohexyl) peroxydicarbonate, dimyristyl peroxydicarbonate, diacetyl peroxydicarbonate, an allyl perester, cumyl peroxyneodecanoate, tert-butyl per-3,5,5-trimethylhexanoate, acetylcyclohexylsulfonyl peroxide, dilauryl peroxide, dibenzoyl peroxide, and tert-amyl perneodecanoate. Particularly suitable polymerization initiators are water-soluble azo initiators, e.g., 2,2'-azobis(2-amidinopropane) dihydrochloride, 2,2'-azobis(N,N'-dimethylene)isobutyramidine dihydrochloride, 2-(carbamoylazo-isobutyronitrile, 2,2'-azobis[2-(2'-imidazolin-2-yl)propane] dihydrochloride, and 4,4'-azobis(4-cyanovaleric acid). The polymerization initiators are used, for example, in amounts of 0.01% to 5%, and preferably 0.05% to 2%, by weight, based on the monomers to be polymerized.

Polymerization initiators also include redox catalysts. In redox catalysts, the oxidizing compound comprises at least one of the above-specified per compounds, and the reducing component comprises, for example, ascorbic acid, glucose, sorbose, ammonium or alkali metal bisulfite, sulfite, thiosulfate, hyposulfite, pyrosulfite, or sulfide, or a metal salt, such as iron (II) ions or sodium hydroxymethylsulfoxylate. The reducing component of the redox catalyst preferably is ascorbic acid or sodium sulfite. Based on the amount of monomers used in the polymerization, about 3 x 10⁻⁶ to about 1 mol% of the reducing component of the redox catalyst system can be used, and about 0.001 to about 5.0 mol% of the oxidizing component of the redox catalyst can be used, for example.

When polymerization is initiated using high energy radiation, the initiator typically comprises a photoinitiator. Photoinitiators include, for example, α-splitters, H-abstracting systems, and azides. Examples of such initiators include, but are not limited to, benzophenone derivatives, such as Michler's ketone; phenanthrene derivatives; fluorene derivatives; anthraquinone derivatives; thioxanthone derivatives; coumarin derivatives; benzoin ethers and derivatives thereof; azo compounds, such as the above-mentioned free-radical formers, substituted hexaarylbisimidazoles, acylphosphine oxides; or mixtures thereof.

Examples of azides include, but are not limited to, 2-(N,N-dimethylamino)ethyl 4-azidocinnamate, 2-(N,N-dimethylamino)ethyl 4-azidonaphthyl ketone, 2-(N,N-dimethylamino)ethyl 4-azidobenzoate, 5-azido-l-naphthyl 2'-(N,N-dimethylamino)-ethyl sulfone, N-(4-sulfonylazidophenyl)maleimide, N-acetyl-4-sulfonylazidoaniline, 4-sulfonyl-azidoaniline, 4-azidoaniline, 4-azidophenacyl bromide, p-azidobenzoic acid, 2,6-bis(p-azidobenzylidene)cyclohexanone, and 2,6-bis(p-azidobenzylidene)-4-methylcyclohexanone. Photoinitiators customarily are used, if at all, in amounts of about 0.01% to about 5%, by weight of the monomers to be polymerized.

As previously stated, the base polymer is partially neutralized. The degree of neutralization preferably is greater than about 25 mol%, more preferably about 50 to about 100 mol%, most preferably about 70 to about 100 mol%, based on monomers containing acid groups.

Useful neutralizing agents for the base polymer include alkali metal bases, ammonia, and/or amines. Preferably, the neutralizing agent comprises aqueous sodium hydroxide, aqueous potassium hydroxide, or lithium hydroxide. However, neutralization also can be achieved using sodium carbonate, sodium bicarbonate, potassium carbonate, or potassium bicarbonate, or other carbonates or bicarbonates, as a solid or as a solution. Primary, secondary, and/or tertiary amines can be used to neutralize the base polymer.

Neutralization of the SAP can be performed before, during, or after polymerization of the monomers in a suitable apparatus for this purpose. The neutralization is performed, for example, directly in a kneader used for polymerization of the monomers.

Polymerization of an aqueous monomer solution, i.e., gel polymerization, is a preferred manufacturing method. In this method, a 10% to 70%, by weight, aqueous solution of the monomers, including the internal crosslinking agent, is neutralized in the presence of a free radical initiator. The solution polymerization is performed at 0°C to 150°C, preferably at 10°C to 100°C, and at atmospheric, superatmospheric, or reduced pressure. The polymerization also can be conducted under a protective gas atmosphere, preferably under nitrogen.

After polymerization, the resulting SAP hydrogel is comminuted and dried, then the resulting dry SAP particles are ground and classified to a predetermined size for an optimum fluid absorption profile. The SAP particles then are surface crosslinked. Surface crosslinking of the SAP particles is optional, however, the SAP particles typically are surface crosslinked. As discussed more fully hereafter, the SAP particles first can be surface crosslinked, then coated with the film-forming polymer and/or wax. Alternatively, surface crosslinking can be performed simultaneously with applying the film-forming polymer and/or wax to the SAP particles. When a surface crosslinking step is omitted, the film-forming polymer and/or wax is applied prior to or during a drying step after an SAP hydrogel is comminuted into particles.

In the optional surface crosslinking process, a multifunctional compound capable of reacting with the functional groups of the SAP is applied to the surfaces of the SAP particles, preferably using an aqueous solution. The aqueous solution also can contain water-miscible organic solvents, like an alcohol, such as methanol, ethanol, or i-propanol; a polyol, like ethylene glycol or propylene glycol; or acetone.

A solution of a surface crosslinking agent is applied to the SAP particles in an amount to wet predominantly only the outer surfaces of the SAP particles, either before, during, or after application of the polymer and/or wax. Surface crosslinking and drying of the SAP particles then is performed, preferably by heating at least the wetted surfaces of the SAP particles.

Typically, the SAP particles are surface treated with a solution of a surface crosslinking agent containing about 0.01% to about 4%, by weight, surface crosslinking agent, and preferably about 0.4% to about 2%, by weight, surface crosslinking agent, in a suitable solvent. The solution can be applied as a fine spray onto the surfaces of freely tumbling SAP particles at a ratio of about 1:0.01 to about 1:0.5 parts by weight SAP particles to solution of surface crosslinking agent. The surface crosslinking agent, if present at all, is present in an amount of 0.001% to about 5%, by weight of the SAP particles, and preferably 0.001% to about 0.5% by weight. To achieve the full advantage of the present invention, the surface crosslinking agent is present in an amount of about 0.001% to about 0.1%, by weight of the SAP particles.

Surface crosslinking and drying of the SAP particles are achieved by heating the surface-treated SAP particles at a suitable temperature, e.g., about 70°C to about 150°C, and preferably about 105°C to about 120°C. Suitable surface crosslinking agents are capable of reacting with acid moieties and crosslinking polymers at the surfaces of the SAP particles.

Nonlimiting examples of suitable surface crosslinking agents include, but are not limited to, an alkylene carbonate, such as ethylene carbonate or propylene carbonate; a polyaziridine, such as 2,2-bishydroxymethyl butanol tris[3-(1-aziridine propionate] or bis-N-aziridinomethane; a haloepoxy, such as epichlorohydrin; a polyisocyanate, such as 2,4-toluene diisocyanate; a di- or polyglycidyl compound, such as diglycidyl phosphonates, ethylene glycol diglycidyl ether, or bischlorohydrin ethers of polyalkylene glycols; alkoxysilyl compounds; polyols such as ethylene glycol, 1,2-propanediol, 1,4-butanediol, glycerol, methyltriglycol, polyethylene glycols having an average molecular weight M_{w} of 200-10,000, di- and polyglycerol, pentaerythritol, sorbitol, the ethoxylates of these polyols and their esters with carboxylic acids or carbonic acid, such as ethylene carbonate or propylene carbonate; carbonic acid derivatives, such as urea, thiourea, guanidine, dicyandiamide, 2-oxazolidinone and its derivatives, bisoxazoline, polyoxazolines, di- and polyisocyanates; di- and poly-N-methylol compounds, such as methylenebis(N-methylolmethacrylamide) or melamine-formaldehyde resins; compounds having two or more blocked isocyanate groups, such as trimethylhexamethylene diisocyanate blocked with 2,2,3,6-tetramethylpiperidin-4-one; and other surface crosslinking agents known to persons skilled in the art.

A solution of the surface crosslinking agent is applied to the surfaces of the SAP particles simultaneously with, or before or after, a dispersion containing the film-forming polymer or wax is applied to the surfaces of the SAP particles. The film-forming polymer or wax typically is applied prior to completion of the surface crosslinking step. In embodiments wherein a film-forming polymer and/or wax is applied to SAP particles that will not be surface crosslinked, the polymer and/or wax is applied after chopping of the SAP hydrogel and prior to completely drying the SAP particles.

A dispersion or slurry containing the film-forming polymer or wax comprises about 5% to about 75%, by weight, of the polymer or wax in a suitable carrier. The polymer or wax is dispersed in a sufficient amount of a carrier to allow the polymer or wax to be readily and homogeneously applied to the surfaces of the SAP particles. The carrier for the polymer or wax can be, but is not limited to, water, an alcohol, or a glycol, such as methanol, ethanol, ethylene glycol, or propylene glycol, and mixtures thereof. Often, the polymer or wax is applied as an emulsion containing the polymer or wax, water, optional organic solvents, emulsifiers, and other ingredients typically used in the preparation of emulsions.

The nonreactive, film-forming polymer or wax applied to the SAP particles is in the form of an aqueous dispersion, emulsion, or suspension. However, the polymer or wax also can be applied from a solution wherein the polymer or wax is dissolved in an organic solvent or a mixture of water and an organic water-miscible solvent.

Additional suitable organic carriers for the film-forming polymer or wax include, but are not limited to, aliphatic and aromatic hydrocarbons, alcohols, ethers, esters, and ketones, for example, n-hexane, cyclohexane, toluene, xylene, methanol, ethanol, i-propanol, ethylene glycol, 1,2-propanediol, glycerol, diethyl ether, methyltriglycol, a polyethylene glycol having an average molecular weight (Mw) of 200-10,000, ethyl acetate, n-butyl acetate, acetone, 2-butanone, and mixtures thereof.

The amount of polymer and/or wax applied to the surfaces of the SAP particles is sufficient to coat the SAP particle surfaces, but not adversely affect the absorption profile of the SAP particles. Accordingly, the amount of polymer applied to the surfaces of the SAP particles is less than about 0.1% of the weight of the SAP particles. Preferably, the amount of polymer applied to the surfaces of the SAP particles is about 0.01% to about 0.08%, by weight of the SAP particles. To achieve the full advantage of the present invention, the amount of polymer applied to the SAP particle surfaces is about 0.02% to about 0.07%, by weight of the SAP particles. The amount of wax applied to the surfaces of the SAP particles is less than about 0.05%, and preferably about 0.01% to less than 0.05%, by weight of the SAP particles.

The polymer and/or wax applied to surfaces of the SAP particles allow for the collection and aggregation of SAP fines generated during SAP manufacture. Accordingly, the problem of fine-sized SAP particle generation is substantially reduced. Furthermore, coated SAP particles of the present invention are not covered with a closed polymer and/or wax film. Rather, the surfaces of SAP particles coated with the polymer and/or wax retain pores of sufficient size and sufficient number such that no adverse effect on the absorption properties of the SAP particles occurs. If the SAP particles are coated with too great of a quantity of polymer or wax, then the number and size of the pores on the particles decrease and an undesirable impairment of SAP absorption property results. In addition, if the amount of polymer or wax applied to surface of the SAP particles is too great, a friable film having a tendency to flake off the SAP particles results. The above-listed amounts of polymer or wax permits the aggregation of fine-sized SAP particles, without adversely affecting the absorption of a fluid by the SAP particles.

After heating the coated SAP particles to form a coating on the SAP particle surfaces, the resulting polymer and/or wax coating on the SAP particle has sufficient adhesive forces to aggregate fine-sized SAP particles to one another or to a larger SAP particle. The adhesive force is sufficient such that SAP fines are not regenerated during subsequent SAP processing or after the SAP particles absorb a fluid and swell.

A film-forming polymer useful in the present invention is different from an SAP, i.e., the polymer does not absorb several times its weight in water and swell. The applied polymer is water insoluble and is nonreactive, i.e., contains no reactive group which can react with the carboxyl groups on the surface of the SAP particles. Suitable polymers for use in the present invention have a glass transition temperature, Tg, of less than about 30°C, preferably less than about 25°C, and more preferably less than about 20°C, and down to at -20°C, for example. The glass transition temperature is the temperature at which an amorphous material, such as a polymer, changes from a brittle vitreous state to a plastic state. The Tg of a polymer is readily determined by persons skilled in the art using standard techniques. Preferred polymers form tackless polymer films between about 0°C and about 80°C.

Useful polymers include homo- and copolymers of vinyl esters, in particular vinyl acetate homopolymers and vinyl acetate copolymers with ethylene, acrylates, maleic acid esters, vinylamides, and/or other vinylacyl derivatives. Homo- and copolymers of acrylic and methacrylic acid esters, such as copolymers of methyl methacrylate, n-butyl acrylate, or 2-ethylhexyl acrylate, also are useful.

Copolymers based on vinyl esters, acrylic acid esters, and methacrylic acid esters comprise comonomers, for example, styrene, butadiene, a vinylamide, an olefinically unsaturated carboxylic acid or derivatives thereof, a vinylphosphonic acid or derivative thereof, or a polyglycol ester of unsaturated acids. Examples of vinylamides include, but are not limited to, N-vinylformamide, N-vinyl-N-methylacetamide, and N-vinylpyrrolidone.

Examples of olefinically unsaturated carboxylic acids are, for example, acrylic acid, methacrylic acid, itaconic acid, and maleic acid. Examples of derivatives of these olefinically unsaturated carboxylic acids are, for example, amides, such as (meth)acrylamide, N-tert-butyl (meth)acrylamide, and N-isopropyl (meth)acrylamide, and the N-methylolamides or ethers of N-methylolamides, hemiamides, and imides of aliphatic amines, as well as acrylonitrile. Examples of derivatives of vinylphosphonic acid are, for example, the mono- and diesters of C₁-C₁₈ alcohols, for example, the methyl, propyl, or stearyl esters.

Glycol esters of unsaturated acids include hydroxyethyl (meth)acrylate or esters of acrylic and methacrylic acid with polyalkylene oxide compounds of the general formula 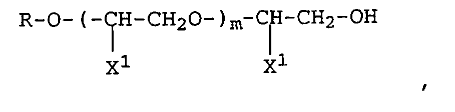 wherein X¹ is hydrogen or methyl, n is 0 to 50, and R is an alkyl, alkaryl, or cycloalkyl C₁-C₂₄ radical, for example, octylphenyl, dodecyl, or nonylphenyl.

Other film-forming polymers are polyacetals, i.e., reaction products of polyvinyl alcohols with aldehydes, such as, for example, butyraldehyde; polyurethane polymers prepared from polyhydric alcohols and isocyanates, for example, prepared from polyester and/or polyether diols and, for example, toluene-2,4- or 2,6-diisocyanate, methylene-4,4-di(phenyl isocyanate), or hexamethylene diisocyanate; polyureas, i.e., polymers prepared from diamines and diisocyanates or by polycondensation of diamines with carbon dioxide, phosgene, carboxylic acid esters (for example, activated diphenyl carbonates), or urea, or by reaction of diisocyanates with water; polysiloxanes, i.e., linear dimethylpolysiloxane having end groups blocked in different ways; polyamides and copolyamides; polyesters, i.e., polymers prepared by ring-opening polymerization of lactones or by condensation of hydroxycarboxylic acids or diols and dicarboxylic acid derivatives; epoxy resins prepared from polyepoxides by addition reactions with suitable curing agents or by polymerization by way of epoxide groups; polycarbonates prepared by reaction of diglycols or bisphenols with phosgene or carbonic acid diesters in condensation or transesterification reactions; and mixtures thereof.

Preferred polymers are homo- and co-polymers of acrylic acid esters and methacrylic acid esters, and polymers based on polyacetals. Mixtures of two or more polymers also can be used. The mixture ratios are noncritical and are judiciously determined by persons skilled in the art to fit the particular circumstances.

The SAP particles also can be coated with a water-insoluble, nonreactive wax to substantially reduce the amount of fine-sized SAP particles during SAP manufacture. Useful waxes have a melting point of about 30°C to about 180°C, preferably about 40°C to about 180°C. To achieve the full advantage of the present invention, the wax has a melting point of about 40°C to about 170°C. Preferred waxes form tackless films up to at least 80°C. Useful waxes also are nonreactive, i.e., they have no reactive groups capable of reacting with carboxyl groups on the surface of the SAP particles.

The following are nonlimiting examples of waxes useful in the present invention. In particular, examples of waxes useful in the present invention include natural waxes, modified natural waxes, semisynthetic waxes, and synthetic waxes. Examples of natural waxes are plant waxes or animal waxes. Plant waxes include, but are not limited to, carnauba wax, candelilla wax, ouricuri wax, sugarcane wax, and retamo wax. Examples of animal waxes include, but are not limited to, insect waxes, e.g., beeswax, ghedda wax, shellac wax, and wool wax. Further examples of natural waxes are fossil waxes, such as petroleum waxes, brown coal (lignite), and peat waxes, e.g., ozokerite, tank-bottom wax, and crude montan wax. Examples of modified natural waxes are waxes obtained by refining, for example, the macro- and microcrystalline paraffin waxes recovered from petroleum distillates or distillation residues, or chemically modified waxes, for example, double-bleached crude montan wax. Examples of semisynthetic waxes include, but are not limited to, acid waxes and ester waxes which can be prepared from montan wax, wax acids which can be prepared by paraffin oxidation, and alcohol waxes and amide waxes. Examples of synthetic waxes include, but are not limited to, hydrocarbon waxes, such as polyolefin waxes and Fischer Tropsch waxes, and synthetic waxes containing oxygen-functional groups. Examples of synthetic waxes containing oxygen-functional groups are acid waxes formed by oxidation of synthetic hydrocarbon waxes or by copolymerization or telomerization of olefins with unsaturated carboxylic acids, ester waxes obtained by esterifying synthetic wax acids with synthetic alcohols and by copolymerizing olefins with unsaturated esters, for example, vinyl acetate, alcohol waxes prepared by oxo synthesis followed by hydrogenation and by hydrogenation of synthetic fatty acids, and amide waxes obtained by reacting synthetic acids with amines. Examples of waxes obtained by oxidation of synthetic hydrocarbon waxes are oxidation products of polyethylene waxes. Preferred waxes for use in accordance with the present invention are refined (i.e., deresinified or bleached) montan waxes and polyolefin waxes.

Particularly preferred waxes for use in accordance with the present invention are polyolefin waxes, such as polyethylene waxes (high pressure polyethylene waxes, low pressure polyethylene waxes, and degradation polyethylene waxes), oxidation products of these polyethylene waxes, waxes based on ethylene-a-olefin copolymers, waxes based on ethylene-vinyl acetate copolymers, waxes based on ethylene-styrene copolymers, waxes based on ethylene-acrylic acid copolymers, and waxes based on wax mixtures of polyethylene waxes with poly(tetrafluoroethylene) waxes.

Mixtures of two or more waxes also can be used. The mixture ratios are noncritical and are judiciously determined by persons skilled in the art according to the particular circumstances. In addition, a mixture of a film-forming polymer and a wax can be used to coat the SAP particles.

In accordance with the present invention, the polymer and/or wax is applied to the SAP particles in a manner such that the polymer and/or wax is uniformly distributed on the surfaces of the SAP particles. Any known method for applying a liquid to a solid can be used, preferably by dispersing a coating liquid into fine droplets, for example, by use of a pressurized nozzle or a rotating disc. Uniform coating of the SAP particles can be achieved in a high intensity mechanical mixer or a fluidized mixer which suspends the SAP particles in a turbulent gas stream. Methods for the dispersion of a liquid onto the surfaces of SAP particles are known in the art, see, for example, U.S. Patent No. 4,734,478, incorporated herein by reference.

Our method of coating the SAP particles is applying the polymer and/or wax and a surface crosslinking agent (if used) simultaneously. Individual components preferably are applied via separate nozzles to avoid any interactions before application to the surfaces of the SAP particles. Another method of coating the base polymer is a sequential addition of the components. The preferred method is a simultaneous application of a surface crosslinking agent and application of the polymer and/or wax. The resulting coated SAP particles then are heated at about 70°C to about 175°C for sufficient time, e.g., about 5 to about 90 minutes, to cure the polymer and/or wax coating.

In accordance with the invention, the present methods substantially reduce the amount of SAP fines generated during SAP manufacture. In particular, the present methods generate less than 2%, and preferably less than 1%, by weight, particles having a particle size of 100 microns or less. More preferably, the methods generate less than 0.5%, by weight, SAP particles having a particle size of 100 microns or less. Accordingly, recycling of SAP fines is substantially reduced, which provides cost savings and improves final SAP performance.

In addition to the film-forming polymer and/or wax, the coating on the SAP particles can include an optional clay. The clay is applied to the SAP particles when the polymer, wax, or surface-crosslinking agent is applied to the SAP particles. The clay can be applied alone or in admixture with the polymer, wax, or optional surface-crosslinking agent.

A clay is present in the coating to provide the benefit of an additional ingredient to assist in agglomerating fine-sized SAP particles. Therefore, a clay is present, if at all, in an amount of about 0.1% to about 1.5%, and preferably about 0.25% to about 1.25%, by weight of the SAP particles. To achieve the full advantage of the present invention, the clay is present in an amount of about 0.4% to about 1.1%, by weight of the SAP particles.

A clay useful in the present invention typically is a nonswelling clay. However, swelling clays also can be used. Suitable nonswelling clays include, without limitation, kaolin minerals (including kaolinite, dickite, and nacrite), serpentine minerals, mica minerals (including illite), chlorite minerals, sepolite, palygorskite, bauxite, and mixtures thereof. Examples of useful swelling clays include, but are not limited to, montmorillonite, i.e., bentonite, beidelite, hectorite, saponite, nontronite, sauconite, and laponite.

To demonstrate the unexpected advantages provided by the method of the present invention, polymer or wax-coated SAP particles were prepared and tested for centrifuge retention capacity (CRC, g/g), absorbency under load (e.g., AUL 0.7 psi, g/g), and particle size distribution. These tests were performed using the following procedures.

### Centrifuge Retention Capacity (CRC)

This test determines the free swelling capacity of a hydrogel-forming polymer. In this method, 0.2000 ± 0.0050 g of dry SAP particles of size fraction 106 to 850 µm are inserted into a teabag. The teabag is placed in saline solution (i.e., 0.9 wt% aqueous sodium chloride) for 30 minutes (at least 0.83 1 (liter) saline solution/1 g polymer). Then, the teabag is centrifuged for 3 minutes at 250 G. The absorbed quantity of saline solution is determined by measuring the weight of the teabag.

### Absorbency Under Load (AUL)

This procedure is disclosed in WO 00/62825, pages 22-23, incorporated herein by reference, using a 230 gram weight for an AUL (0.70 psi). AUL (0.01 psi) and AUL (0.9 psi) are determined using the same procedure using an appropriate weight, e.g., a 317 gram weight for an AUL (0.9 psi).

### Particle Size Distribution (PSD)

Particle size distribution is determined as set forth in U.S. Patent No. 5,061,259, incorporated herein by reference. In summary, a sample of SAP particles is added to the top of a series of stacked sieves. The sieves are mechanically shaken for a predetermined time, then the amount of SAP particles on each sieve is weighed. The percent of SAP particles on each sieve is calculated from the initial sample weight of the SAP sample.

### Example 1

| Base SAP particles: | CRC=40 g/g |
|---|---|
| PSD: >850 µm: | 0.1% |
| 500-850 µm: | 33.8% |
| 300-500 µm: | 32.6% |
| 150-300 µm: | 26.5% |
| 106-150 µm: | 5.7% |
| 45-106 µm: | 1.3% |
| <45 µm: | 0.0%. |

| | |
|---|---|
| The SAP particles¹⁾ (40 kg) were coated with 2 kg of deionized water, 2 kg of propylene glycol, 0.025 kg of polymer²⁾, and 0.04 kg of ethylene glycol diglycidyl ether (EGDGE). The SAP particles then were cured at 120°C for 60 minutes. | |

**Finished product performance:**

| CRC=30.3 g/g | |
|---|---|
| PSD: >850 µm: | 0.3% |
| 500-850 µm: | 36.0% |
| 300-500 µm: | 32.8% |
| 150-300 µm: | 25.8% |
| 106-150 µm: | 4.1% |

| | |
|---|---|
| 45-106 µm: | 1.0% |
| <45 µm: | 0.0% |

| | |
|---|---|
| ¹⁾ The SAP particles were a crosslinked polyacrylic acid having a 75% neutralization; and ²⁾ Added as an emulsion containing 50.5%, by weight of a methyl methacrylate/butyl acrylate copolymer, to provide 0.025 kg of the polymer. | |

**Comparative Example 1**

| Base SAP particles: | CRC=40 g/g |
|---|---|
| PSD: >850 µm: | 0.1% |
| 500-850 µm: | 33.8% |
| 300-500 µm: | 32.6% |
| 150-300 µm: | 26.5% |
| 106-150 µm; | 5.7% |
| 45-106 µm: | 1.3% |
| <45 µm: | 0.0% |

| | |
|---|---|
| The SAP particles¹⁾ (40 kg) were coated with 2 kg of deionized water, 2 kg of propylene glycol, and 0.04 kg of EGDGE. The SAP particles then were cured at 120°C for 60 minutes. | |

**Finished product performance:**

| CRC=30.1 g/g | |
|---|---|
| AUL (0.7 psi)=17.5 g/g | |
| PSD: >850 µm: | 1.2% |
| 500-850 µm: | 2.92% |
| 300-500 µm: | 28.2% |
| 150-300 µm: | 24.2% |
| 106-150 µm; | 5.8% |
| 45-106 µm: | 9.3% |
| <45 µm: | 2.1% |

Example 1 and Comparative Example 1 show that coating SAP particles with a polymer substantially reduces the amount of fine-sized SAP particles generated during the surface crosslinking process, without adversely affecting fluid absorption properties.

A series of polymers and a wax were tested for an ability to reduce the amount of fine-sized SAP particles generated during SAP manufacture. Three of the four polymers were styrene/butadiene copolymers of different concentrations. The fourth polymer was a methyl methacrylate/butyl acrylate (MMA/BA) copolymer. The objective of the test was to minimize the amount 106 micron and smaller SAP particles generated during the surface crosslinking process.

| | |
|---|---|
| Polymer 1: | Solid content=50.5% MMA/BA copolymer; |
| Polymer 2: | Solid content=70.8% styrene/butadiene copolymer; |
| Polymer 3: | Solid content=50.8% styrene/butadiene copolymer; |
| Polymer 4: | Solid content=52.5% styrene/butadiene copolymer. |

### Example 2

To polyacrylic acid SAP particles (DN=75) was added 4.2% (by weight, based on the weight of the SAP particles) of a mixture of a 1/1 ratio of deionized (DI) water and propylene glycol that further contained 0.1% Polymer 1 latex. The mixture was applied by spraying onto 1,000 grams of SAP particles fluidized in a Lodige mixer. After applying Polymer 1, the SAP particles were heated to 120°C and held for one hour. The initial SAP particles polymer had a 106 micron and smaller particle content of 20%. After application of Polymer 1, the particle distribution was measured and found to have 0% of particles having a size less than 106 micron. The CRC of the coated SAP particles was 32.7 g/g and the SAUL was 62 (0.01 AUL=50 g/g and 0.9 AUL=12 g/g). Target properties of SAP particles without added Polymer 1 are 30 g/g CRC and 64 SAUL. The finished product whiteness was 36.

### Example 3

To polyacrylic acid SAP particles (DN=75) was added 4.2% (by weight based on the weight of the SAP particles) of a mixture of a 1/1 ratio of DI water and propylene glycol that further contained 0.1% EGDGE, 0.05% Polymer 1, and 1% of kaolin clay. The mixture first was sheared in a Waring Blender at the "high speed" condition for three minutes to disperse Polymer 1 more uniformly. The mixture then was applied by spraying onto 1,000 grams of SAP particles that were fluidized in a Lodige mixer. After applying the mixture, the SAP particles were heated to 120°C and held for one hour. After application of the polymer/kaolin clay coating, the mean PSD was measured and determined to be 43% higher than the control (330 µm versus 281 µm) indicating a shift in the particle distribution (PSD) towards the coarse end and a reduction of the amount of fine-sized SAP particles.

### Example 4

To polyacrylic acid SAP particles (DN=75) was added a 4% mixture (by weight based on the weight of the SAP particles) of a 3/1 ratio of DI water and propylene glycol that also contains 4% EGDGE, 0.058% micronized polyethylene wax, and 1% of kaolin clay. This mixture was sheared in a Waring Blender at the "high speed" condition for three minutes to disperse the wax more uniformly. The mixture then was applied by spraying onto 1,000 grams of SAP particles fluidized in a Lodige mixer. After applying the mixture, the SAP particles were heated to 120°C and held for one hour. The initial SAP particles had a less than 45 micron content of 3.5%. After wax application, the particle distribution was measured and found to have 2% of the particles less than 45 microns.

The SAP particles of the present invention are useful as absorbents for water and other aqueous fluids, and can be used as an absorbent component in hygiene articles, such as diapers, tampons, and sanitary napkins. The SAP particles also can be used in the following applications, for example: storage, packaging, transportation as a packaging material for water-sensitive articles, for example, flower transportation, and shock protection; food sector for transportation of fish and fresh meat, and the absorption of water and blood in fresh fish and meat packs; water treatment, waste treatment and water removal; cleaning; and agricultural industry in irrigation, retention of meltwater and dew precipitates, and as a composting additive.

Particularly preferred applications for the present SAP particles include medical uses (wound plaster, water-absorbent material for burn dressings or for other weeping wounds, rapid dressings for injuries, rapid uptake of body fluid exudates for later analytical and diagnostic purposes), cosmetics, carrier material for pharmaceuticals and medicaments, rheumatic plaster, ultrasound gel, cooling gel, thickeners for oil/water or water/oil emulsions, textile (gloves, sportswear, moisture regulation in textiles, shoe inserts, synthetic fabrics), hydrophilicization of hydrophobic surfaces, chemical process industry applications (catalyst for organic reactions, immobilization of large functional molecules (enzymes), heat storage media, filtration aids, hydrophilic component in polymer laminates, dispersants, liquefiers), and building construction (sealing materials, systems or films that self-seal in the presence of moisture, and fine-pore formers in sintered building materials or ceramics).

The present invention also provides for use of the SAP particles in an absorption core of hygienic articles. Hygiene articles include, but are not limited to, incontinence pads and incontinence briefs for adults, diapers for infants, catamenial devices, bandages, and similar articles useful for absorbing body fluids.

Hygiene articles, like diapers, comprise (a) a liquid pervious topsheet; (b) a liquid impervious backsheet; (c) a core positioned between (a) and (b) and comprising 10% to 100% by weight of the present SAP particles, and 0% to 90% by weight of a hydrophilic fiber material; (d) optionally a tissue layer positioned directly above and below said core (c); and (e) optionally an acquisition layer positioned between (a) and (c).

Obviously, many modifications and variations of the invention as hereinbefore set forth can be made without departing from the spirit and scope thereof and, therefore, only such limitations should be imposed as are indicated by the appended claims.

## Claims

1. A superabsorbent polymer particle comprising a base polymer having a surface coating comprising less than 0.1%, by weight of the particle, of a water-insoluble, nonreactive, film-forming polymer; less than 0.05%, by weight of the particle, of a wax; or both.

2. The superabsorbent polymer particle of claim 1 wherein the particle is surface crosslinked.

3. The superabsorbent polymer particle of claim 1 wherein the base polymer contains a plurality of pendant neutralized and unneutralized carboxylic acid groups.

4. The superabsorbent polymer particle of claim 1 wherein the base polymer has a degree of neutralization from about 25 to about 100.

5. The superabsorbent polymer particle of claim 1 wherein the base polymer comprises acrylic acid, methacrylic acid, or a mixture thereof.

6. The superabsorbent polymer particle of claim 1 wherein the polymer is present on surfaces of the base polymer in an amount of about 0.01% to about 0.08%, by weight of the particle.

7. The superabsorbent polymer particle of claim 1 wherein the polymer is present on surfaces of the base polymer in an amount of about 0.02% to about 0.07%, by weight of the particle.

8. The superabsorbent polymer particle of claim 1 wherein the wax is present on surfaces of the base polymer in an amount of about 0.01% to less than 0.05%, by weight of the particle.

9. The superabsorbent polymer particle of claim 1 wherein the coating comprises a film-forming polymer.

10. The superabsorbent polymer particle of claim 9 wherein the film-forming polymer comprise a homopolymer or a copolymer of a vinyl ester, an acrylic acid ester, a methacrylic acid ester, a polyacetal, a polyurethane, a polysiloxane, a polyester, an epoxy resin, a polycarbonate, or mixtures thereof.

11. The superabsorbent polymer particle of claim 1 wherein the film-forming polymer has a glass transition temperature of less than 30°C.

12. The superabsorbent polymer particle of claim 1 wherein the coating comprises a wax.

13. The superabsorbent polymer particle of claim 12 wherein the wax is selected from the group consisting of a natural wax, a modified natural wax, a semisynthetic wax, a synthetic wax, and mixtures thereof.

14. The superabsorbent polymer particle of claim 12 wherein the wax is selected from the group consisting of a polyolefin wax, a montan wax, a fossil wax, a peat wax, a micro- or macrocrystalline paraffin wax, an acid wax, an ester wax, an alcohol wax, an amide wax, a plant wax, an animal wax, and mixtures thereof.

15. The superabsorbent polymer particle of claim 1 wherein the surface coating further comprises about 0.1% to about 1.5%, by weight of the particle, of a clay.

16. The superabsorbent polymer particle of claim 1 wherein the base polymer comprises a polyacrylic acid.

17. The superabsorbent polymer particle of claim 16 wherein the film-forming polymer comprises a styrene/butadiene copolymer, a methyl methacrylate/butyl acrylate copolymer, or a mixture thereof.

18. The superabsorbent polymer particle of claim 16 wherein the wax comprises a micronized polyethylene wax, a montan wax, a polyethylene wax, or mixtures thereof.

19. The superabsorbent polymer particle of claim 16 wherein the base polymer is surface crosslinked.

20. A method of preparing a superabsorbent polymer particle of claim 1 comprising:
(a) providing base polymer particles;
(b) providing a polymer and a wax;
(c) applying the polymer or the wax, or both, to surfaces of the base polymer particles;
(d) optionally applying a surface crosslinking agent to the surfaces of the base polymer; and
(e) heating the coated base polymer resulting from steps (c) and (d) at a sufficient temperature and for a sufficient time to provide a dry polymer or wax coating on the base polymer particles.

21. The method of claim 20 wherein heating step (e) is performed at 70°C to about 175°C for about 5 minutes to about 90 minutes.

22. The method of claim 20 wherein step (c) is performed prior to step (d).

23. The method of claim 20 wherein step (c) is performed after step (d).

24. The method of claim 20 wherein step (c) and step (d) are performed simultaneously.

25. The method of claim 20 wherein the superabsorbent polymer particles have a particle size distribution wherein less than 2%, by weight, of the particles have a particle size of 100 microns or less.

26. The method of claim 20 wherein the superabsorbent polymer particles have a film-forming polymer coating.

27. The method of claim 20 wherein the superabsorbent polymer particles have a wax coating.

28. The method of claim 20 further comprising the step of applying 0.1% to 1.5% of a clay, by weight of the base polymer particles, to surfaces of the base polymer particles prior to step (e).

29. A method of preparing superabsorbent polymer particles having a water-insoluble, non-reactive, film-forming polymer or wax coating comprising:
(a) providing surface-crosslinked base polymer particles;
(b) providing a polymer or a wax, or both;
(c) applying the polymer in an amount of less than 0.1% by weight of the base polymer particles, the wax in an amount of less than 0.05%, by weight of the base polymer particles, or both, to surfaces of the base polymer particles;
(d) heating the coated base polymer resulting from step (c) at a sufficient temperature and for a sufficient time to provide a dry polymer or wax coating on the surface-crosslinked base polymer particles.

30. The method of claim 29 wherein the superabsorbent polymer particles have a particle size distribution wherein less than 2%, by weight, of the particles have a particle size of 100 microns or less.

31. The method of claim 29 wherein the superabsorbent polymer particles have a film-forming polymer coating.

32. The method of claim 29 wherein the superabsorbent polymer particles have a wax coating.

33. The method of claim 29 further comprising the step of applying 0.1% to 1.5% of a clay, by weight of the base polymer particles, to surfaces of the base polymer particles prior to step (d).

34. A hygienic article having a core, said core comprising superabsorbent polymer particles of claim 1.

35. The hygienic article of claim 34 wherein said article is selected from the group consisting of a diaper, an incontinence pad, an incontinence brief, a catamenial device, and a bandage.

36. A hygiene article comprising (a) a liquid pervious topsheet; (b) a liquid impervious backsheet; (c) a core positioned between (a) and (b) and comprising (i) 10% to 100% by weight of the superabsorbent polymer particles of claim 1 and (ii) 0% to 90% by weight of a hydrophilic fiber material; (d) optionally a tissue layer positioned directly above and below said core (c); and (e) optionally an acquisition layer positioned between (a) and (c).

37. The article of claim 36 wherein the hygiene article is a diaper.

## Patentansprüche

1. Superabsorberpartikel, umfassend ein Basispolymer mit einer Oberflächenbeschichtung, umfassend weniger als 0,1 Gew.-%, bezogen auf das Gewicht des Partikels, eines wasserunlöslichen, nicht reaktionsfähigen, filmbildenden Polymers, weniger als 0,05 Gew.-%, bezogen auf das Gewicht des Partikels, eines Wachses oder beides.

2. Superabsorberpartikel nach Anspruch 1, bei dem das Partikel oberflächenvernetzt ist.

3. Superabsorberpartikel nach Anspruch 1, bei dem das Basispolymer mehrere anhängige neutralisierte und nicht neutralisierte Carbonsäuregruppen enthält.

4. Superabsorberpartikel nach Anspruch 1, bei dem das Basispolymer einen Neutralisationsgrad von etwa 25 bis etwa 100 aufweist.

5. Superabsorberpartikel nach Anspruch 1, bei dem das Basispolymer Acrylsäure, Methacrylsäure oder eine Mischung davon umfaßt.

6. Superabsorberpartikel nach Anspruch 1, bei dem das Polymer in einer auf das Gewicht des Partikels bezogenen Menge von etwa 0,01% bis etwa 0,08% auf Oberflächen des Basispolymers vorliegt.

7. Superabsorberpartikel nach Anspruch 1, bei dem das Polymer in einer auf das Gewicht des Partikels bezogenen Menge von etwa 0,02% bis etwa 0,07% auf Oberflächen des Basispolymers vorliegt.

8. Superabsorberpartikel nach Anspruch 1, bei dem das Wachs in einer auf das Gewicht des Partikels bezogenen Menge von etwa 0,01% bis weniger als 0,05% auf Oberflächen des Basispolymers vorliegt.

9. Superabsorberpartikel nach Anspruch 1, bei dem die Beschichtung ein filmbildendes Polymer umfaßt.

10. Superabsorberpartikel nach Anspruch 9, bei dem das filmbildende Polymer ein Homopolymer oder ein Copolymer eines Vinylesters, eines Acrylsäureesters, eines Methacrylsäureesters, ein Polyacetal, ein Polyurethan, ein Polysiloxan, einen Polyester, ein Epoxidharz, ein Polycarbonat oder Mischungen davon umfaßt.

11. Superabsorberpartikel nach Anspruch 1, bei dem das filmbildende Polymer eine Glasübergangstemperatur von weniger als 30°C aufweist.

12. Superabsorberpartikel nach Anspruch 1, bei dem die Beschichtung ein Wachs umfaßt.

13. Superabsorberpartikel nach Anspruch 12, bei dem das Wachs ausgewählt ist aus der Gruppe, bestehend aus einem Naturwachs, einem modifizierten Naturwachs, einem halbsynthetischen Wachs, einem synthetischen Wachs und Mischungen davon.

14. Superabsorberpartikel nach Anspruch 12, bei dem das Wachs ausgewählt ist aus der Gruppe, bestehend aus einem Polyolefinwachs, einem Montanwachs, einem fossilen Wachs, einem Torfwachs, einem mikro- oder makrokristallinen Paraffinwachs, einem Säurewachs, einem Esterwachs, einem Alkoholwachs, einem Amidwachs, einem Pflanzenwachs, einem Tierwachs und Mischungen davon.

15. Superabsorberpartikel nach Anspruch 1, bei dem die Oberflächenbeschichtung ferner bezogen auf das Gewicht des Partikels etwa 0,1% bis etwa 1,5% eines Tons umfaßt.

16. Superabsorberpartikel nach Anspruch 1, bei dem das Basispolymer eine Polyacrylsäure umfaßt.

17. Superabsorberpartikel nach Anspruch 16, bei dem das filmbildende Polymer ein Styrol-Butadien-Copolymer, ein Methylmethacrylat-Butylacrylat-Copolymer oder eine Mischung davon umfaßt.

18. Superabsorberpartikel nach Anspruch 16, bei dem das Wachs ein mikronisiertes Polyethylenwachs, ein Montanwachs, ein Polyethylenwachs oder Mischungen davon umfaßt.

19. Superabsorberpartikel nach Anspruch 16, bei dem das Basispolymer oberflächenvernetzt ist.

20. Verfahren zur Herstellung eines Superabsorberpartikels gemäß Anspruch 1, bei dem man
(a) Basispolymerpartikel bereitstellt,
(b) ein Polymer und ein Wachs bereitstellt,
(c) das Polymer oder das Wachs oder beides auf Oberflächen der Basispolymerpartikel aufträgt,
(d) gegebenenfalls ein Oberflächenvernetzungsmittel auf die Oberflächen des Basispolymers aufträgt und
(e) das aus den Schritten (c) und (d) resultierende beschichtete Basispolymer bei einer ausreichenden Temperatur ausreichend lange erhitzt, um eine trockene Polymer- oder Wachsschicht auf den Basispolymerpartikeln bereitzustellen.

21. Verfahren nach Anspruch 20, bei dem das Erhitzen (e) bei 70°C bis etwa 175°C etwa 5 Minuten bis etwa 90 Minuten lang erfolgt.

22. Verfahren nach Anspruch 20, bei dem man Schritt (c) vor Schritt (d) durchführt.

23. Verfahren nach Anspruch 20, bei dem man Schritt (c) nach Schritt (d) durchführt.

24. Verfahren nach Anspruch 20, bei dem man Schritt (c) und Schritt (d) gleichzeitig durchführt.

25. Verfahren nach Anspruch 20, bei dem die Superabsorberpartikel eine Partikelgrößenverteilung aufweisen, bei der weniger als 2 Gew.-% der Partikel eine Partikelgröße von 100 Mikron und weniger aufweisen.

26. Verfahren nach Anspruch 20, bei dem die Superabsorberpartikel mit einem filmbildenden Polymer beschichtet werden.

27. Verfahren nach Anspruch 20, bei dem die Superabsorberpartikel mit einem Wachs beschichtet werden.

28. Verfahren nach Anspruch 20, bei dem man ferner bezogen auf das Gewicht der Basispolymerpartikel 0,1% bis 1,5% eines Tons auf Oberflächen der Basispolymerpartikel vor Schritt (e) aufträgt.

29. Verfahren zur Herstellung von Superabsorberpartikeln mit einer Beschichtung aus einem wasserunlöslichen, nicht reaktionsfähigen, filmbildenden Polymer oder Wachs, bei dem man
(a) oberflächenvernetzte Basispolymerpartikel bereitstellt,
(b) ein Polymer oder ein Wachs oder beides bereitstellt,
(c) das Polymer in einer Menge von weniger als 0,1%, bezogen auf das Gewicht der Basispolymerpartikel, das Wachs in einer Menge von weniger als 0,05%, bezogen auf das Gewicht der Basispolymerpartikel, oder beides auf Oberflächen der Basispolymerpartikel aufträgt,
(d) das aus Schritt (c) resultierende beschichtete Basispolymer bei einer ausreichenden Temperatur ausreichend lange erhitzt, um eine trockene Polymer-oder Wachsbeschichtung auf den oberflächenvernetzten Basispolymerpartikeln bereitzustellen.

30. Verfahren nach Anspruch 29, bei dem die Superabsorberpartikel eine Partikelgrößenverteilung aufweisen, bei der weniger als 2 Gew.-% der Partikel eine Partikelgröße von 100 Mikron und weniger aufweisen.

31. Verfahren nach Anspruch 29, bei dem die Superabsorberpartikel mit einem filmbildenden Polymer beschichtet werden.

32. Verfahren nach Anspruch 29, bei dem die Superabsorberpartikel mit einem Wachs beschichtet werden.

33. Verfahren nach Anspruch 29, bei dem man ferner bezogen auf das Gewicht der Basispolymerpartikel 0,1% bis 1,5% eines Tons auf Oberflächen der Basispolymerpartikel vor Schritt (d) aufträgt.

34. Hygieneartikel mit Kern, wobei der Kern Superabsorberpartikel gemäß Anspruch 1 umfaßt.

35. Hygieneartikel nach Anspruch 34, bei dem der Artikel ausgewählt ist aus der Gruppe, bestehend aus einer Windel, einer Inkontinenzeinlage, einem Inkontinenzhöschen, einem Monatshygieneprodukt und einem Verband.

36. Hygieneartikel, umfassend (a) eine obere flüssigkeitsdurchlässige Abdeckung; (b) eine untere flüssigkeitsundurchlässige Schicht; (c) einen zwischen (a) und (b) befindlichen Kern, umfassend (i) 10 bis 100 Gew.-% der Superabsorberpartikel gemäß Anspruch 1 und (ii) 0 bis 90 Gew.-% eines hydrophilen Fasermaterials; (d) gegebenenfalls eine sich unmittelbar oberhalb und unterhalb des Kerns (c) befindende Tissueschicht und (e) gegebenenfalls eine zwischen (a) und (c) sich befindende Aufnahmeschicht.

37. Artikel nach Anspruch 36, bei dem es sich bei dem Hygieneartikel um eine Windel handelt.

## Revendications

1. Particule de polymère superabsorbant comprenant un polymère de base comportant un revêtement de surface comprenant un polymère filmogène insoluble dans l'eau, non réactif, représentant moins de 0,1 % en poids de la particule ; une cire représentant moins de 0,05 % en poids de la particule ; ou les deux.

2. Particule de polymère superabsorbant selon la revendication 1, la particule étant réticulée en surface.

3. Particule de polymère superabsorbant selon la revendication 1, dans laquelle le polymère de base contient une pluralité de groupes pendants d'acide carboxylique neutralisé et non neutralisé.

4. Particule de polymère superabsorbant selon la revendication 1, dans laquelle le polymère de base a un degré de neutralisation d'environ 25 à environ 100.

5. Particule de polymère superabsorbant selon la revendication 1, dans laquelle le polymère de base comprend l'acide acrylique, l'acide méthacrylique, ou un mélange de ceux-ci.

6. Particule de polymère superabsorbant selon la revendication 1, dans laquelle le polymère est présent sur les surfaces du polymère de base dans une quantité d'environ 0,01 % à environ 0,08 % en poids de la particule.

7. Particule de polymère superabsorbant selon la revendication 1, dans laquelle le polymère est présent sur les surfaces du polymère de base dans une quantité d'environ 0,02 % à environ 0,07 % en poids de la particule.

8. Particule de polymère superabsorbant selon la revendication 1, dans laquelle la cire est présente sur les surfaces du polymère de base dans une quantité d'environ 0,01 % à moins de 0,05 % en poids de la particule.

9. Particule de polymère superabsorbant selon la revendication 1, dans laquelle le revêtement comprend un polymère filmogène.

10. Particule de polymère superabsorbant selon la revendication 9, dans laquelle le polymère filmogène comprend un homopolymère ou un copolymère d'un ester vinylique, d'un ester d'acide acrylique, d'un ester d'acide méthacrylique, un polyacétal, un polyuréthane, un polysiloxane, un polyester, une résine époxyde, un polycarbonate, ou des mélanges de ceux-ci.

11. Particule de polymère superabsorbant selon la revendication 1, dans laquelle le polymère filmogène a une température de transition vitreuse inférieure à 30 °C.

12. Particule de polymère superabsorbant selon la revendication 1, dans laquelle le revêtement comprend une cire.

13. Particule de polymère superabsorbant selon la revendication 12, dans laquelle la cire est sélectionnée dans le groupe constitué d'une cire naturelle, d'une cire naturelle modifiée, d'une cire semi-synthétique, d'une cire synthétique, et de mélanges de celles-ci.

14. Particule de polymère superabsorbant selon la revendication 12, dans laquelle la cire est sélectionnée dans le groupe constitué d'une cire polyoléfinique, d'une cire de lignite, d'une cire fossile, d'une cire de tourbe, d'une cire de paraffine microcristalline ou macrocristalline, d'une cire d'acide, d'une cire d'ester, d'une cire d'alcool, d'une cire amide, d'une cire végétale, d'une cire animale, et de mélanges de celles-ci.

15. Particule de polymère superabsorbant selon la revendication 1, dans laquelle le revêtement de surface comprend en outre une quantité d'argile représentant environ 0,1 % à environ 1,5 % en poids de la particule.

16. Particule de polymère superabsorbant selon la revendication 1, dans laquelle le polymère de base comprend un acide polyacrylique.

17. Particule de polymère superabsorbant selon la revendication 16, dans laquelle le polymère filmogène comprend un copolymère de styrène/butadiène, un copolymère de méthacrylate de méthyle/acrylate de butyle, ou un mélange de ceux-ci.

18. Particule de polymère superabsorbant selon la revendication 16, dans laquelle la cire comprend une cire de polyéthylène micronisée, une cire de lignite, une cire de polyéthylène, ou des mélanges de celles-ci.

19. Particule de polymère superabsorbant selon la revendication 16, dans laquelle le polymère de base est réticulé en surface.

20. Procédé de préparation d'une particule de polymère superabsorbant selon la revendication 1, comprenant :
(a) la fourniture de particules de polymère de base ;
(b) la fourniture d'un polymère et d'une cire ;
(c) l'application du polymère ou de la cire, ou des deux, sur les surfaces des particules de polymère de base ;
(d) éventuellement l'application d'un agent de réticulation superficielle sur les surfaces du polymère de base ; et
(e) le chauffage du polymère de base revêtu résultant des étapes (c) et (d) à une température suffisante et pendant suffisamment de temps pour produire un revêtement sec de polymère ou de cire sur les particules de polymère de base.

21. Procédé selon la revendication 20, dans lequel l'étape de chauffage (e) est exécutée à une température de 70 °C à environ 175 °C pendant environ 5 minutes à environ 90 minutes.

22. Procédé selon la revendication 20, dans lequel l'étape (c) est exécutée avant l'étape (d).

23. Procédé selon la revendication 20, dans lequel l'étape (c) est exécutée après l'étape (d).

24. Procédé selon la revendication 20, dans lequel l'étape (c) et l'étape (d) sont exécutées simultanément.

25. Procédé selon la revendication 20, dans lequel les particules de polymère superabsorbant ont une distribution granulométrique dans laquelle moins de 2 % en poids des particules ont une taille de particule de 100 microns ou moins.

26. Procédé selon la revendication 20, dans lequel les particules de polymère superabsorbant comportent un revêtement de polymère filmogène.

27. Procédé selon la revendication 20, dans lequel les particules de polymère superabsorbant comportent un revêtement de cire.

28. Procédé selon la revendication 20, comprenant en outre l'étape d'application de 0,1 % à 1,5 % d'une argile, ce pourcentage étant exprimé en poids relativement aux particules de polymère de base, sur les surfaces des particules de polymère de base avant l'étape (e).

29. Procédé de préparation de particules de polymère superabsorbant comportant un revêtement de polymère filmogène insoluble dans l'eau, non réactif, ou de cire, comprenant :
(a) la fourniture de particules de polymère de base réticulées en surface ;
(b) la fourniture d'un polymère ou d'une cire, ou des deux ;
(c) l'application du polymère dans une quantité représentant moins de 0,1 % en poids des particules de polymère de base, de la cire dans une quantité représentant moins de 0,05 % en poids des particules de polymère de base, ou des deux, sur les surfaces des particules de polymère de base ;
(d) le chauffage du polymère de base revêtu résultant de l'étape (c) à une température suffisante et pendant suffisamment de temps pour produire un revêtement sec de polymère ou de cire sur les particules de polymère de base réticulées en surface.

30. Procédé selon la revendication 29, dans lequel les particules de polymère superabsorbant ont une distribution granulométrique dans laquelle moins de 2 % en poids des particules ont une taille de particule de 100 microns ou moins.

31. Procédé selon la revendication 29, dans lequel les particules de polymère superabsorbant comportent un revêtement de polymère filmogène.

32. Procédé selon la revendication 29, dans lequel les particules de polymère superabsorbant comportent un revêtement de cire.

33. Procédé selon la revendication 29, comprenant en outre l'étape d'application de 0,1 % à 1,5 % d'une argile, ce pourcentage étant exprimé en poids relativement aux particules de polymère de base, sur les surfaces des particules de polymère de base avant l'étape (d).

34. Article hygiénique comportant une partie centrale, ladite partie centrale comprenant des particules de polymère superabsorbant selon la revendication 1.

35. Article hygiénique selon la revendication 34, ledit article hygiénique étant sélectionné dans le groupe constitué d'une couche pour bébé, d'une couche d'incontinence, d'une culotte d'incontinence, d'un article cataménial, et d'un bandage.

36. Article hygiénique comprenant (a) une couche supérieure perméable aux liquides ; (b) une couche de fond imperméable aux liquides ; (c) une partie centrale positionnée entre (a) et (b) et comprenant (i) de 10 % à 100 % en poids des particules de polymère superabsorbant selon la revendication 1, et (ii) de 0 à 90 % en poids d'un matériau fibreux hydrophile ; (d) éventuellement une couche de papier-mouchoir positionnée directement au-dessus et en dessous de ladite partie centrale (c) ; et (e) éventuellement une couche d'acquisition positionnée entre (a) et (c).

37. Article selon la revendication 36, l'article hygiénique étant une couche pour bébé.
